# EUROPEAN PATENT APPLICATION

(11) **EP 0 856 333 A2**
(43) Date of publication of application: **05.08.1998**
(21) Application number: 98100848.5
(22) Date of filing: 19.01.1998
(51) Int. Cl.: A61N 1/372, A61B 5/00

(54) **Transtelephone system for monitoring and programming implantable cardiac pacemakers and defibrillators**

(30) Priority: 30.01.1997 IT MI970178
(71) Applicant: Ela Medical S.P.A., 20090 Segrate (Milano) (IT)
(72) Inventor: Rognoni, Giorgio, 28100 Novara (IT); Bottazzi, Marco, 27042 - Bressana Bottarone (PV) (IT); Voltan, Gianfranco, 20020 Arese (Milano) (IT)
(74) Representative: Petruzziello, Aldo

(57) **Abstract**

A transtelephone system for monitoring and programming implantable cardiac pacemakers and defibrillators comprising at least one remote station (1) connected to a programming head (10) of a cardiac pacemaker capable of receiving the operating parameters of an implanted cardiac pacemaker or defibrillator and a local station (2) connected by means of telephone lines (8,8',20), for transmission of the parameters thus obtained from said at least one remote station (1) to said local station (2).

## Description

The present invention relates to a transtelephone support system for monitoring and programming implantable cardiac pacemakers and defibrillators.

What is meant by transtelephone support system is a telematic system which, besides offering cardiac pacemaker clinics a telephone help line, provides a further connection which allows the parameters on which to intervene, read on implanted cardiac pacemakers and defibrillators, to be viewed at the same time by medical personnel and by a technical expert such as a bioengineer, for example.

The support systems used to date provide either solely a telephone help line or, when required, the presence of a bioengineer.

These systems have the obvious drawback of offering a service limited to use of the telephone line only and require the physical presence of a bioengineer if the medical personnel consider it appropriate.

The object of the present invention is to eliminate the above drawbacks, providing a transtelephone support system which, though ensuring a complete service, does not require the physical presence of the bioengineer, allowing an obvious reduction in intervention time, and the possibility of offering support and care in a plurality of centres, even located in different cities.

These and other objects are achieved with a transtelephone support system for programming cardiac pacemakers, according to the invention, which has the characteristics listed in claim 1.

Preferred embodiments of the invention emerge from the dependent claims.

Substantially, the system according to the invention proposes a structure that makes it possible to provide complete remote support, allowing the data for analysis to be viewed both by the physician and by the bioengineer.

Further characteristics of the invention will be made clearer by the detailed description that follows, referring to a purely exemplary and therefore non-limiting embodiment thereof, illustrated in the appended drawings, in which:
Figure 1 is a connection structure for transtelephone support in which are shown both a local station in the assisting centre and a generic remote station in the assisted centre connected to each other by both a telephone line and a transtelephone data transmission line, as well as a line for possible transmission of a surface ECG signal (electrocardiographic tracing),
Figure 2 shows a local station in detail,
Figure 3 shows a generic remote station in detail,
Figure 4 shows the flow chart for a of transtelephone support session for programming a cardiac pacemaker,
Figure 5 shows a star diagram of a transtelephone support system according to the invention, with a local supporting station and different remote stations situated in different supported hospitals.

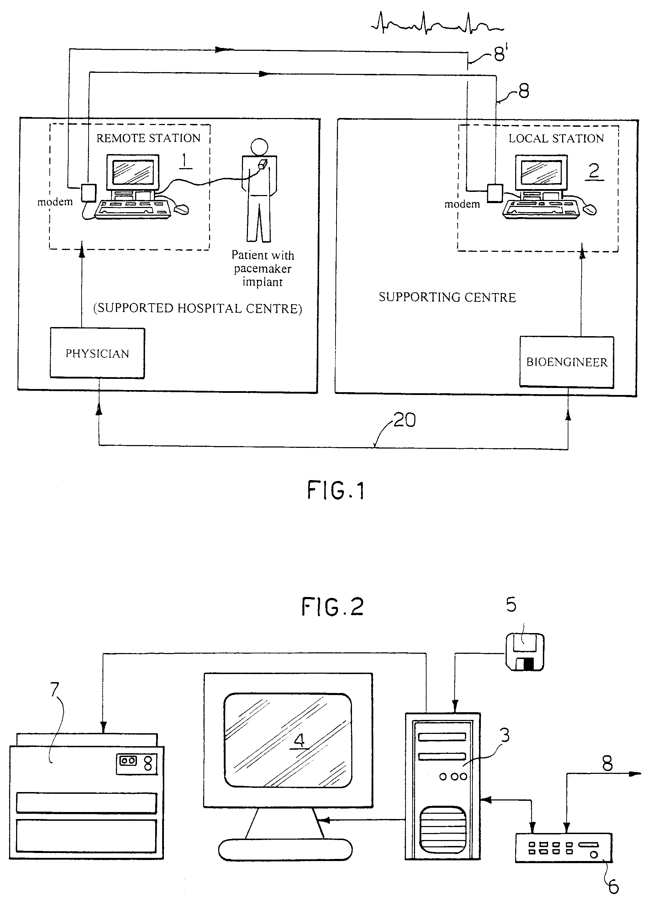

In figure 1 the transtelephone support system according to the invention is shown in general, where a remote station 1 is shown at a hospital where the service for reading and monitoring of the adjustment data of implantable cardiac pacemakers and defibrillators is carried out, and a local station 2, at the supporting centre where the transtelephone support service is carried out. It shows how the medical personnel can interact with the bioengineer through a telephone line 20 possibly using the toll-free line made available by the supporting centre. Furthermore, after the data have been read from the patient's cardiac pacemaker they are transferred, possibly together with a surface ECG signal (electrocardiographic tracing), through telephone lines 8 and 8', from the remote station 1 where the medical personnel operate, to the local station 2 for support, including visual support, to the bioengineer of the patient's general condition to guide and recommend the choice of parameters that might be modified.

In Figure 2 a local station 2 at a supporting centre is shown in detail, in its constituent parts, comprising a computer 3 in which a program 5 for management of the data to be received from the remote station 1 and the transtelephone support conditions in connection with a monitor 4 for displaying them and printer 7 for reproducing them as hard copy. Furthermore, the computer 3 is connected to a modem 6 in turn connected to a through telephone line 8 to allow connection to different remote stations 1.

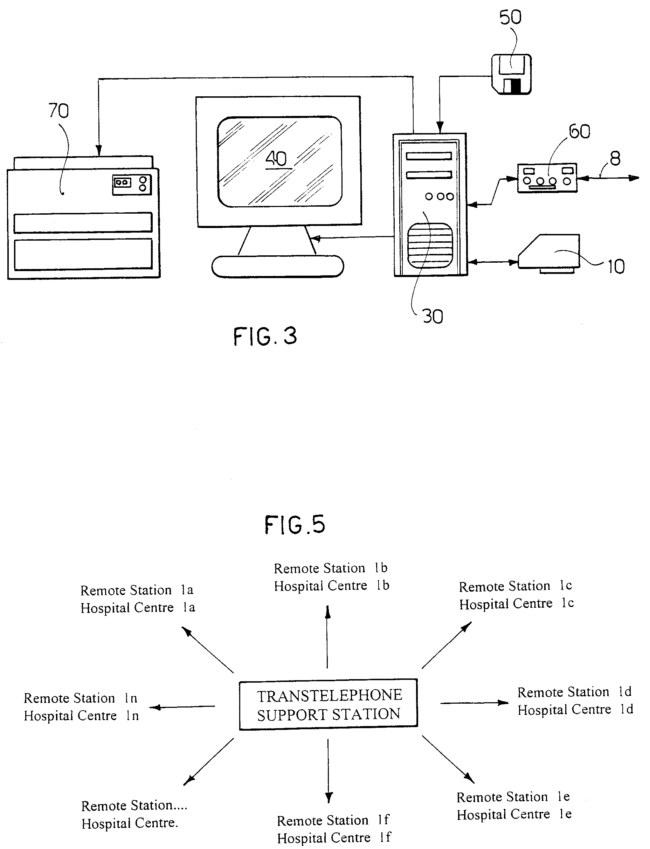

In Figure 3, on the other hand, a remote station 1, at a supported centre, is shown in detail that has a similar structure to that of the local station 2, with a computer 30, with a program 50 installed to interrogate the cardiac pacemaker and for management of the data to be sent to the local station 2 and of the transtelephone support connections, a monitor 40, a printer 70 and a modem 60 connected to a through telephone line 8.

For reading of the parameters to be examined, the programming head of the cardiac pacemaker 10 which is set up to record the data of the cardiac pacemaker implanted in the patient is connected to the computer 30.

After having been displayed on the monitor 40 of the remote station 1, these data are sent to the local station 2 via the through telephone line 8, to be displayed on the monitor 4 thereof.

In addition, if the telephone line 20 is not available for an audio connection, the possibility is foreseen of transmitting messages in real time by means of the through telephone line 8.

With the system according to the invention, therefore, both the bioengineer and the medical personnel have the same information displayed; thus the bioengineer can recommend any changes that the medical personnel can decide to make to the cardiac pacemaker, as though he were present.

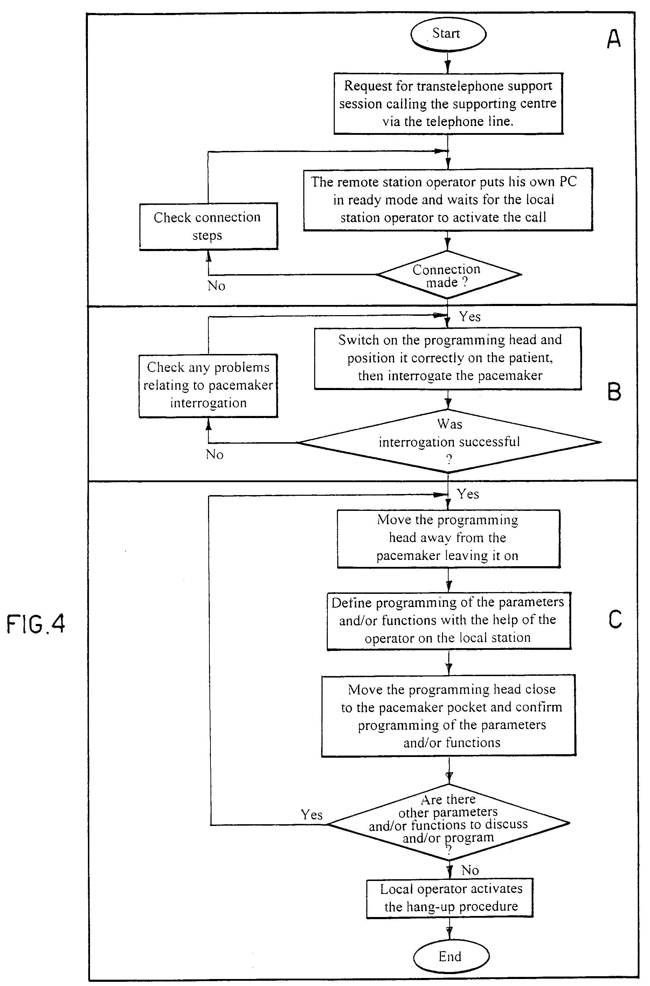

In Figure 4 a flow chart is shown for the entire remote support session for programming of a cardiac pacemaker in which the sequence for making the connection between the remote station 1 and the local station 2, which is repeated until the connection has been made, is illustrated in part A; part B is that concerning interrogation of the cardiac pacemaker by the programming head 10, which is repeated until it has been completed; in part C the sequences are shown for first defining and then carrying out programming of the parameters or functions of the cardiac pacemaker.

For safety reasons, programming of the programming head of the cardiac pacemaker 10 is carried out when this is not in contact with the cardiac pacemaker, and to prevent any programming confirmation maneuver on the cardiac pacemaker by the local station 2. When programming has been agreed with the bioengineer, the programming head is brought close to the cardiac pacemaker for confirmation of the parameters decided.

Figure 5 shows the structure of a star-type connection of the local station 2 for transtelephone support with different remote stations 1a, 1b, 1c etc., in respective supported centers, which highlights the versatility of the remote assistance system according to the invention which can provide connections with different hospitals through telephone line connections, ensuring ample coverage even for stations that are physically distant from the local station 2.

## Claims

1. A transtelephone support system for controlling and programming implantable cardiac pacemakers and defibrillators comprising at least one remote station (1) at a supported center connected to a programming head (10) of a cardiac pacemaker, used to receive the operating parameters of an implanted cardiac pacemaker or defibrillator, and a local station (2) at a supporting centre connected by means of telephone lines (8, 8', 20).

2. A system according to claim 1, characterized in that an audio connection by means of a telephone line (20) is provided between said supported centre and said supporting centre.

3. A system according to claim 1 or 2, characterized in that the parameters acquired from the cardiac pacemaker by said at least one remote station (1) are transmitted to said local station (2) by means of a through telephone line (8).

4. A system according to claim 3, characterized in that said through telephone line (8) is also used for transmitting messages.

5. A system according to any one of the preceding claims, characterized in that said local station (2) comprises a computer (3) that contains a program (5) for management of data and of said system on the station (2), a monitor (4) to display the data to be processed, a modem (6) for the connection to said one through telephone line (8).

6. A system according to any one of the preceding claims, characterized in that said remote station (1) comprises a computer (30), which contains a program (50) for interrogating the cardiac pacemaker, for management of the data and of said system on the station (1), a monitor (40) to display the data for processing, a modem (60) for connection to a through telephone line (8) and a programming head for cardiac pacemakers or defibrillators (10).

7. A system according to any one of the preceding claims, characterized in that it also provides for transmission of a surface ECG signal from said at least one remote station (1) to the supporting station (2).

8. A system according to claim 7, characterized in that said surface ECG signal is transmitted on a separate telephone line (8').
